# EUROPEAN PATENT SPECIFICATION

(11) **EP 3 020 401 B1**
(45) Date of publication and mention of the grant of the patent: **13.11.2019**
(21) Application number: 14822133.6
(22) Date of filing: 09.07.2014
(51) Int. Cl.: A61K 31/423, A61P 1/16, A61P 43/00

(54) **PEMAFIBRATE FOR USE IN THE TREATMENT OF NON-ALCOHOLIC FATTY LIVER DISEASE**
PEMAFIBRAT ZUR BEHANDLUNG DER NICHTALKOHOLISCHEN FETTLEBERERKRANKUNG
PÉMAFIBRAT DESTINÉ AU TRAITEMENT DE LA STÉATOSE HÉPATIQUE NON ALCOOLIQUE

(30) Priority: 10.07.2013 JP 2013144643
(43) Date of publication of application: 18.05.2016
(73) Proprietor: Kowa Company, Ltd., Nagoya-shi, Aichi 460-8625 (JP)
(72) Inventor: SHIBATA, Haruki, Higashimurayama-shi Tokyo 189-0022 (JP); TAKIZAWA, Toshiaki, Higashimurayama-shi Tokyo 189-0022 (JP)
(74) Representative: Adam, Holger
(86) International application number: PCT/JP2014/068253
(87) International publication number: WO 2015/005365

(56) References cited:
- EP-A1- 1 661 890
- EP-A1- 2 573 081
- WO-A1-2005/023777
- WO-A1-2011/145340
- WO-A2-2008/040548
- WO-A2-2011/118976
- JP-A- 2013 522 359
- Y. YAMAZAKI ET AL.: 'Design and synthesis of highly potent and selective human peroxisome proliferator-activated receptor a agonists' BIOORGANIC & MEDICINAL CHEMISTRY LETTERS vol. 17, no. 16, 24 May 2007, pages 4689 - 4693, XP029094754

## Description

The present invention relates to (R)-2-[3-[[N-(benzoxazol-2-yl)-N-3-(4-methoxy-phenoxy)propyl]aminomethyl]phenoxy]butyric acid or a salt thereof, or a solvate thereof for use in the prophylaxis and/or treatment of nonalcoholic fatty liver disease and to (R)-2-[3-[[N-(benzoxazol-2-yl)-N-3-(4-methoxy-phenoxy)propyl]aminomethyl]phenoxy]butyric acid or a salt thereof, or a solvate thereof for use in the prophylaxis and/or treatment of nonalcoholic steatohepatitis.

### Background Art

Attention has been paid to metabolic syndrome, which is a generic term for what are called lifestyle-related diseases such as type 2 diabetes. Metabolic syndrome is defined as a state in which visceral obesity is combined two or more of hyperglycemia, hypertension, and dyslipidemia. Metabolic syndrome is thought to promote arteriosclerosis and to increase the risk of the development of myocardial infarction, cerebral infarction, and the like.

Nonalcoholic fatty liver disease (hereinafter also referred to as NAFLD) is a fatty liver disorder that occurs in the absence of alcohol consumption and other clear causes (such as viruses and autoimmune disorders). Recently, NAFLD is recognized as a phenotype of metabolic syndrome in the liver. Other various pathogenesis impairing fat metabolism or mitochondrial metabolism has been reported. NAFLD encompasses diseases ranging from simple steatosis, which is caused only by fat deposition in hepatocytes and has a relatively benign prognosis, to nonalcoholic steatohepatitis (hereinafter also referred to as NASH), which is relatively severe and can lead to liver tissue fibrosis, liver cirrhosis, and hepatocarcinoma (Non Patent Document 1).

As the development mechanism of NASH, "two hit theory" (Day et al.) is widely known (Non Patent Document 2). According to the theory, an imbalance between caloric intake and expenditure and storage of lipid in hepatocytes due to metabolic disorder based on insulin resistance are involved in the formation process of the fatty liver (1st hit). As the 2nd hit, an increase of oxidative stress due to energy metabolic load and an activation of the innate immune system accompanied thereby play an important role in the progression of fatty liver to NASH. Kupffer (CD68 positive) cells, which are immunocompetent cells in the liver, are hepatic resident macrophages and reported to increase in number in NASH patients (Non Patent Document 3). Macrophages are also reported to increase in number in the liver of NASH model mice (Non Patent Document 4). Experimental removal of Kupffer cells is also reported to suppress high-fat diet-induced adiposity in the liver, and Kupffer cells are suggested to play an important role in the development of NASH (Non Patent Document 5) .

The therapy of NASH is, in principle, an improvement in lifestyle based on the diet and exercise therapy for lifestyle-related diseases such as obesity, diabetes, dyslipidemia, and hypertension. Inreality, however, lifestyle improvements are difficult to achieve, and thus drug therapies targeting insulin resistance, oxidative stress, lipid metabolism abnormality, or hypertension, which are considered as important factors for the development of NASH are provided. As a therapeutic drug, an insulin resistance-improving drug such as thiazolidine derivatives (e.g., pioglitazone and rosiglitazone), which are ligands of nuclear receptor PPARγ associated with the potentiation of insulin sensitivity or a biguanide drug (e.g., metformin), i.e., an insulin resistance-improving drug is used. Further, an antioxidant such as vitamin E is used alone or in combination with vitamin C. Furthermore, as a therapeutic agent for lipid metabolism abnormality, fibrates (e.g., fenofibrate and bezafibrate) which is a PPARα agonist and a statin formulation, probucol, or the like is expected. As a therapeutic agent for hypertension, an angiotensin II type 1 receptor antagonist (ARB) is expected. Particularly, fibrates or statins are expected from the viewpoint of their anti-inflammatory activities. However, there are few reports on studies with high level of evidence such as amelioration of fatty liver disorder symptoms, and no highly-recommended therapies have been established yet. Since the number of individuals affected with metabolic syndrome continues to increase worldwide, the number of NASH patients is expected to increase in the future, and it is desired to establish a method of treatment of NASH (Non Patent Documents 1 and 6).

Meanwhile, Patent Document 1 discloses a compound represented by the following formula (1): 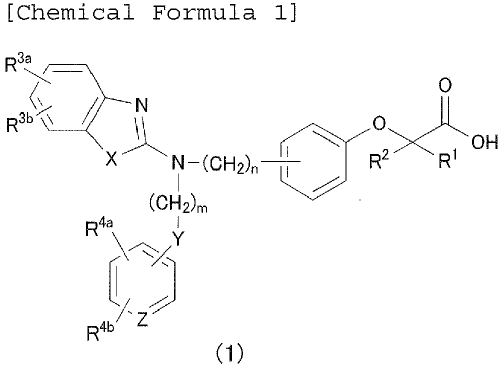 wherein R¹ and R², which may be the same or different from each other, and each represents a hydrogen atom, a methyl group, or an ethyl group; R^{3a}, R^{3b}, R^{4a}, and R^{4b}, which may be the same or different from one another, and each represents a hydrogen atom, a halogen atom, a nitro group, a hydroxyl group, a C₁₋₄ alkyl group, a trifluoromethyl group, a C₁₋₄ alkoxy group, a C₁₋₄ alkylcarbonyloxy group, a di-C₁₋₄ alkylamino group, a C₁₋₄ alkylsulfonyloxy group, a C₁₋₄ alkylsulfonyl group, a C₁₋₄ alkylsulfinyl group, or a C₁₋₄ alkylthio group, wherein R^{3a} and R^{3b} or R^{4a} and R^{4b} may bind to each other to form an alkylenedioxy group; X represents an oxygen atom, a sulfur atom, or N-R⁵, wherein R⁵ represents a hydrogen atom, a C₁₋₄ alkyl group, a C₁₋₄ alkylsulfonyl group, or a C₁₋₄alkyloxycarbonyl group; Y represents an oxygen atom, a S(O)₁ group, wherein 1 is a number from 0 to 2, a carbonyl group, a carbonylamino group, an aminocarbonyl group, a sulfonylamino group, an aminosulfonyl group, or a NH group; Z represents CH or N; n represents a number from 1 to 6; and m represents a number from 2 to 6, or a salt thereof, or a solvate thereof.

Patent Document 1 discloses that the compound, a salt thereof , or a solvate thereof selectively activates PPARα and is useful as a drug for preventing and/or treating, without causing obesity or increase in body weight, hyperlipidemia, arteriosclerosis, diabetes, complications of diabetes (such as diabetic nephropathy), inflammation, and heart diseases in mammals including humans. However, Patent Document 1 does not disclose or suggest what effects these compounds have on NAFLD, specifically, NASH with more serious conditions.

### Citation List

### Patent Document

Patent Document 1: WO 2005/023777 A1

### Non Patent Documents

Non Patent Document 1: Nugent C. et al., Nat. Clin. Pract. Gastroenterol. Hepatol., 4(8), 432-41 (2007)
Non Patent Document 2: Day CP. et al, Gastroenterology, 114(4), 842-5 (1998)
Non Patent Document 3 : Park JW. et al., J. Gastroenterol. Hepatol. , 22, 491-7 (2007)
Non Patent Document 4: Yoshimatsu M. et al., Int. J. Exp. Path., 85, 335-43 (2004)
Non Patent Document 5: Stienstra R. et al., Hepatology, 51(2), 511-22 (2010)
Non Patent Document 6: Uto H., et al., SAISHIN IGAKU vol. 63, no. 9, 1683-7 (2008)

Yamazaki et al. discloses that a combination of benzoxazole, phenoxyalkyl side chain, and phenoxybutyric acids was identified as a highly potent and selective human peroxisome proliferator-activated receptor α (PPARα) agonist (see Bioorganic & Medicinal Chemistry Letters, Vol. 17, Issue 16, 15 August 2007, pages 4689-4693).

WO 2011/118976 A2 provides a pharmaceutical composition for the prevention and treatment of a non-alcoholic fatty liver disease (NAFLD), containing an active ingredient selected from the group consisting of Compound 1 represented by formula 1, sitagliptin, vildagliptin, linagliptin or a pharmaceutically acceptable salt thereof.

EP 1 661 890 A1 relates to a PPAR activating compound which selectively activates, among peroxisome proliferator-activated receptors (PPARs), α-type PPAR (i.e., PPARα), and is useful as a drug for preventing and/or treating pathological conditions including hyperlipidemia, arteriosclerosis, diabetes, complications of diabetes, inflammation, and heart diseases.

None of these documents discloses in one embodiment the features of the claimed invention.

### Summary of the Invention

### Problems to be Solved by the Invention

The number of individuals affected with metabolic syndrome continues to increase worldwide, and the number of NAFLD patients, specifically, NASH patients with more serious conditions is expected to increase in the future. However, there are few reports on studies with high level of evidence on NAFLD therapy, and at present, no highly-recommended therapies have been established yet. An object of the present invention is to provide a novel prophylactic or therapeutic agent for nonalcoholic fatty liver disease, which is useful for preventing or treating NAFLD, specifically, NASH.

### Means for Solving the Problems

In order to find compounds useful for the prevention and/or treatment of nonalcoholic fatty liver disease (NAFLD), specifically, nonalcoholic steatohepatitis (NASH) with serious conditions, the present inventors have investigated compounds useful for prevention and/or treatment by using LDL receptor knockout mice and KK-A^{y} mice fed with an MCD (methionine-choline-deficient) diet, which are NASH model animals. As a result, the present inventors have surprisingly found that the compound disclosed as Example 85 in said Patent Document 1 listed above is effective in suppressing ballooning of hepatocytes and fat deposition and reducing the number of Kupffer cells and thus useful for preventing and/or treating NAFLD, and have accomplished the present invention based on the finding.

The present invention relates to (R)-2-[3-[[N-(benzoxazol-2-yl)-N-3-(4-methoxy-phenoxy)propyl]aminomethyl]phenoxy]butyric acid or a salt thereof, or a solvate thereof for use in the prophylaxis and/or treatment of nonalcoholic fatty liver disease.

The present invention also relates to (R)-2-[3-[[N-(benzoxazol-2-yl)-N-3-(4-methoxy-phenoxy)propyl]aminomethyl]phenoxy]butyric acid or a salt thereof, or a solvate thereof for use in the prophylaxis and/or treatment of nonalcoholic steatohepatitis.

The present disclosure provides a prophylactic and/or therapeutic agent for nonalcoholic fatty liver disease, including, as an active ingredient, (R)-2-[3-[[N-(benzoxazol-2-yl)-N-3-(4-methoxyphenoxy)propyl ]aminomethyl]phenoxy]butyric acid (hereinafter also referred to as Compound A) or a salt thereof, or a solvate thereof.

More specifically, the present disclosure relates the following items (1) to (2).
(1) A prophylactic and/or therapeutic agent for nonalcoholic fatty liver disease, including, as an active ingredient, (R)-2-[3-[[N-(benzoxazol-2-yl)-N-3-(4-methoxyphenoxy)propyl ]aminomethyl]phenoxy]butyric acid or a salt thereof, or a solvate thereof.
(2) The prophylactic and/or therapeutic agent according to item (1), wherein the nonalcoholic fatty liver disease is nonalcoholic steatohepatitis.

### Effects of the Invention

The present invention provides (R)-2-[3-[[N-(benzoxazol-2-yl)-N-3-(4-methoxy-phenoxy)propyl]aminomethyl]phenoxy]butyric acid or a salt thereof, or a solvate thereof for use in the prophylaxis and/or treatment of nonalcoholic fatty liver disease and (R)-2-[3-[[N-(benzoxazol-2-yl)-N-3-(4-methoxy-phenoxy)propyl]aminomethyl]phenoxy]butyric acid or a salt thereof, or a solvate thereof for use in the prophylaxis and/or treatment of nonalcoholic steatohepatitis. Particularly for NASH with serious conditions, there are few reports on therapeutic agents for NASH with high level of evidence, and no highly recommended therapies have been established yet. Since the number of NASH patients is expected to increase worldwide in the future, it is desired to establish a method of treatment for NASH. The present invention provides preferably therefore a prophylactic and/or therapeutic agent capable of suppressing hepatic fat deposition or ballooning of hepatocytes, reducing the number of Kupffer cells in the liver, and being effective for NASH with high severity.

### Brief Description of Drawings

FIG. 1 is a graph showing ballooning of hepatocytes upon the administration of Compound A (0.5 mg/kg) according to the present invention or fenofibrate (100 mg/kg).
FIG. 2 is a graph showing CD68 positive area in liver upon the administration of Compound A (0.5 mg/kg) according to the present invention or fenofibrate (100 mg/kg).
FIG. 3 is a graph showing steatosis score upon the administration of Compound A (0.25 mg/kg) according to the present invention or bezafibrate (60 mg/kg).

### Modes for Carrying Out the Invention

(R)-2-[3-[[N-(benzoxazol-2-yl)-N-3-(4-methoxyphenoxy)pr opyl] aminomethyl] phenoxy] butyric acid (Compound A) for use in the present invention can be produced by, for example, the method described in WO2005/023777A1. Compound A can also be formulated into preparations using the method described in the literatures .

A salt or a solvate of Compound A may also be used in the present invention. The salt or solvate can be produced by conventional methods.

The salt of Compound A may be any pharmacologically acceptable salt. Examples of the salt include alkali metal salts such as sodium and potassium salts; alkaline earth metal salts such as calcium and magnesium salts; organic base salts such as ammonium and trialkylamine salts; mineral acid salts such as hydrochlorides and sulfates; and organic acid salts such as acetates.

The solvates of Compound A or the solvate of the salt of Compound A include hydrates, alcohol solvates (e.g., ethanol solvates).

The compounds represented by Compound A have an asymmetric carbon atom and therefore include R and S optical isomers. Such isomers are all encompassed within the scope of the present disclosure.

CD68 positive Kupffer cells (macrophages in the liver) are known to increase in number in NASH (see Non Patent Document 3). As shown in the examples below, Compound A significantly reduces the number of Kupffer cells and ballooning of hepatocytes in LDL receptor knockout mice fed with a high-fat, high-cholesterol diet (Western diet), which are NASH model animals, and also significantly reduces hepatic fat deposition in KK-A^{y} mice fed with an MCD diet, which are also NASH model animals. Therefore, Compound A according to the present invention or a salt thereof, or a solvate thereof is useful as a prophylactic and/or therapeutic agent for nonalcoholic fatty liver disease, specifically, nonalcoholic steatohepatitis with high severity, in mammals including humans.

The prophylactic and/or therapeutic agent of the present invention encompasses a pharmaceutical composition including an active ingredient for prophylaxis and a pharmaceutically acceptable carrier, a pharmaceutical composition including an active ingredient for treatment and a pharmaceutically acceptable carrier, and a pharmaceutical composition including an active ingredient for prophylaxis and treatment and a pharmaceutically acceptable carrier.

The agent of the present invention for suppressing fat deposition in the liver encompasses a pharmaceutical composition including an active ingredient for suppressing an increase in fat deposition in the liver and a pharmaceutically acceptable carrier, a pharmaceutical composition including an active ingredient for reducing fat deposition in the liver and a pharmaceutically acceptable carrier, and a pharmaceutical composition including an active ingredient for suppressing an increase in fat deposition in the liver and reducing fat deposition in the liver and a pharmaceutically acceptable carrier.

The agent of the present invention for reducing the number of Kupffer cells in the liver encompasses a pharmaceutical composition including an active ingredient for suppressing an increase in the number of Kupffer cells in the liver and a pharmaceutically acceptable carrier, a pharmaceutical composition including an active ingredient for reducing the number of Kupffer cells increased in the liver and a pharmaceutically acceptable carrier, and a pharmaceutical composition including an active ingredient for suppressing an increase in the number of Kupffer cells in the liver and reducing the number of Kupffer cells increased in the liver and a pharmaceutically acceptable carrier.

The prophylactic and/or therapeutic agent of the present invention and the pharmaceutical composition of the present invention includes, as an active ingredient, a compound represented by Compound A or a salt thereof, or a solvate thereof . As shown in the examples below, the active ingredient according to the present invention is useful as a prophylactic and/or therapeutic agent for nonalcoholic fatty liver disease, specifically, nonalcoholic steatohepatitis with high severity, in mammals including humans. As shown in the examples below, the active ingredient according to the present invention is also useful as an agent for suppressing fat deposition in the liver of mammals including humans or as an agent for reducing the number of Kupffer cells in the liver of mammals including humans.

In the present invention, Compound A or a salt thereof, or a solvate thereof may be formulated alone or with any other pharmacologically acceptable carrier into tablets, capsules, granules, powders, lotions, ointments, injections, suppositories, or other dosage forms. These preparations can be produced by known methods. For example, preparations for oral administration can be produced using any appropriate combination of solubilizing agents such as gum tragacanth, gum arabic, sucrose fatty acid ester, lecithin, olive oil, soybean oil, and PEG400; excipients such as starch, mannitol, and lactose; binders such as carboxymethyl cellulose sodium and hydroxypropyl cellulose; disintegrators such as crystalline cellulose and carboxymethyl cellulose calcium; lubricants such as talc and magnesium stearate; and flow improvers such as light anhydrous silicic acid.

In the present invention, Compound A or a salt thereof, or a solvate thereof is administered orally or parenterally. Although the dose of the medicament of the present invention depends on the weight, age, sex, condition, and other characteristics of patients, generally 0.01 to 1,000 mg, preferably 0.1 to 100 mg of Compound A is administered to an adult once or in two or three divided doses per day.

### Examples

Hereinafter, the present invention will be more specifically described with reference to examples.

### Example 1: Effects in LDL receptor knockout mice NASH model

Effects on Western diet-fed LDL receptor knockout mice (Non Patent Document 4), which are known to develop fatty liver and hepatic inflammation, characteristic symptoms of NASH were examined.

This experiment was conducted using (R)-2-[3-[[N-(benzoxazol-2-yl)-N-3-(4-methoxyphenoxy)propyl ]aminomethyl]phenoxy]butyric acid (Compound A), which is disclosed as Example 85 in Patent Document 1 listed above.

### 1) Animals used:

Male LDL receptor knockout mice (B6.129S7-Ldlr<tm1Her>/J, Jackson Laboratories) were used in the experiment. Beginning at around 8 weeks of age, the mice were freely fed with Teklad Custom Research Diet (TD. 88137, Harlan Teklad) as a Western diet for 13 weeks to develop NASH.

### 2) Grouping:

After 1 week of the Western diet feeding, the mice were subjected to blood sampling and weighing. The mice were divided into a control group (0.5% methyl cellulose aqueous solution), a Compound A 0.5 mg/kg administration group, and a fenofibrate 100 mg/kg administration group in such a way that there was no difference in plasma lipid level and weight between the groups.

### 3) Drug administration:

A 0.5% methyl cellulose aqueous solution and drug solutions were orally administered in an amount of 5 mL/kg weight once a day to the control group, the Compound A administration group, and the fenofibrate administration group, respectively. The administration period was 12 weeks.

### 4) Observation and analytical method:

After the administration was completed, the livers were removed from the mice under pentobarbital sodium (50 mg/kg) anesthesia. The livers were fixed with paraformaldehyde and then subjected to the preparation of hematoxylin-eosin stained samples and immunostained samples for CD68 detection. In a blind study setting, using the hematoxylin-eosin stained samples, ballooning of hepatocytes was scored based on the following criteria (Kleiner et al. Hepatology 41, 1313-21, 2005).
No balloon cells: 0
Few balloon cells: 1
Many balloon cells or ballooning: 2
The CD68 positive area in liver was measured with an image analysis system (WinROOF) in a blind study.

The statistical analysis was performed using Stat Preclinica (Takumi Information Technology Inc.) (Ver. 1.1). Dunnett's test (N = 14-15) was used for comparison with the control group, in which a significant difference with p < 0.05 is indicated by the symbol *, and a significant difference with p < 0.001 is indicated by the symbol ***.

### 5) Results

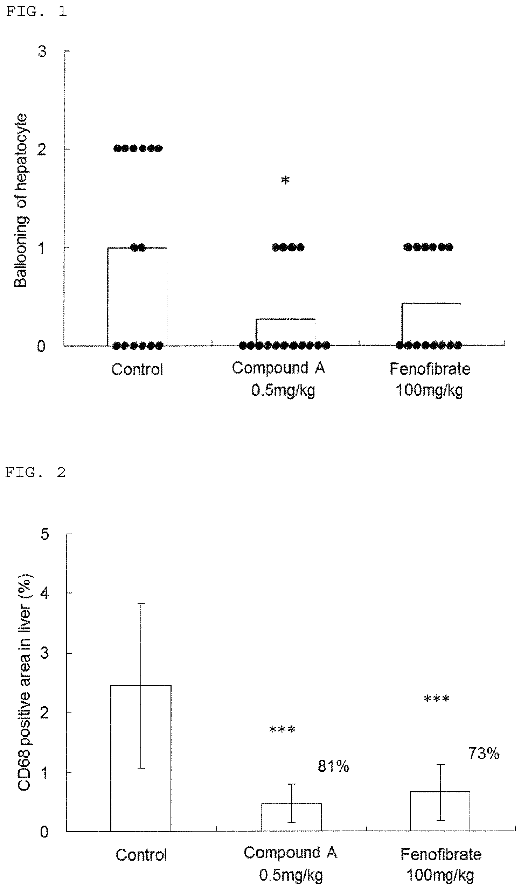

As shown in FIG. 1, significant suppression of ballooning of hepatocytes was observed in the Compound A 0.5 mg/kg administration group. A tendency to suppress ballooning was observed in the fenofibrate 100 mg/kg administration group, but it was not statistically significant. As shown in FIG. 2, the Compound A 0.5 mg/kg administration group showed a significant decrease in CD68 positive area in liver (reduction rate 81%) . The fenofibrate 100 mg/kg administration group also showed a decrease in CD68 positive area in liver, but its reduction rate was 73%, which was less effective than in the Compound A administration group. These results show that Compound A suppresses ballooning of hepatocytes and Kupffer cell-positive area (symptoms of NASH) more strongly than fenofibrate, a PPARα agonist.

### Example 2: Effects in NASH model using KK-A^{y} mice fed with methionine-choline-deficient diet

Effects on experimental animals, KK-A^{y} mice fed with a methionine-choline-deficient diet (MCD diet), which are known to develop fatty liver, a characteristic symptom of NASH (Nakano S. et al., Hepatol Res., 38(10), 1026-39, 2008) were examined.

As in Example 1, this experiment was conducted using (R)-2-[3-[[N-(benzoxazol-2-yl)-N-3-(4-methoxyphenoxy)propyl ]aminomethyl]phenoxy]butyric acid (Compound A), which is disclosed as Example 85 in Patent Document 1 listed above.

### 1) Animals used:

Male KK-AY mice (KK-A^{y}/TaJc1, CLEA Japan, Inc.) were used in the experiment. Beginning at around 12 weeks of age, the mice were freely fed with an MCD diet for 16 weeks to develop NASH.

### 2) Grouping:

The mice were divided into a normal diet group, a control group (fed with an MCD diet), a Compound A 0.25 mg/kg administration group, and a bezafibrate 60 mg/kg administration group in such a way that there was no difference in weight between the groups.

### 3) Drug administration:

The doses were mixed in the feed. The control group was fed with an MCD diet containing no drug. The Compound A administration group was fed with an MCD diet containing 0.00026% of Compound A. The bezafibrate administration group was fed with an MCD diet containing 0.06% of bezafibrate. The administration period was 16 weeks.

### 4) Observation and examination method

After the administration was completed, the livers were removed from the mice under pentobarbital sodium (50 mg/kg) anesthesia. The livers were fixed with paraformaldehyde and then subjected to the preparation of hematoxylin-eosin stained samples. In a blind study setting, evaluation of steatosis scores was performed. The grade of fat deposition was observed with 100 times magnification. Depending on the grade, the fatty liver was scored on a scale of 0 to 3 based on the following criteria.
Less than 5%: 0
5% to less than 33%: 1
33% to less than 66%: 2
66% or more: 3

The statistical analysis was performed using Stat Preclinica (Takumi Information Technology Inc.) (Ver. 1.1). Dunnett's test (N = 4-9) was used for comparison with the control group, in which a significant difference with p < 0.05 is indicated by the symbol *, and a significant difference with p < 0.001 is indicated by the symbol ***.

### 5) Results:

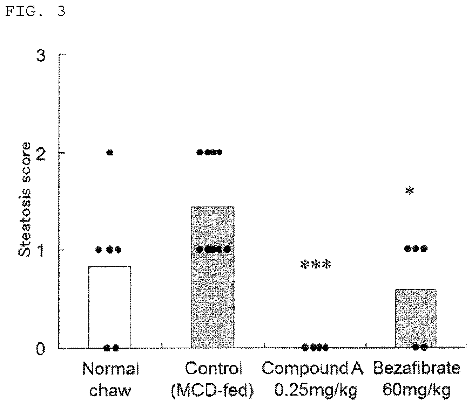

As shown in FIG. 3, the Compound A 0.25 mg/kg administration group showed a steatosis score of 0 for all mice, in other words, almost complete disappearance of fat deposition. In the bezafibrate 60 mg/kg administration group, fat deposition was suppressed to nearly the same extent as in the normal diet group, but not to such an extent that fat deposition almost completely disappeared as in the Compound A administration group. These results show that Compound A suppresses fat deposition, a symptom of NASH, more strongly than bezafibrate, a PPARα agonist.

Thus, Examples 1 and 2 demonstrate that Compound A according to the present invention is very useful as a prophylactic and/or therapeutic agent for nonalcoholic steatohepatitis (NASH) and for nonalcoholic fatty liver disease (NAFLD).

### Industrial Applicability

The present invention provides a low molecular weight, prophylactic and/or therapeutic agent for nonalcoholic fatty liver disease based on new findings that Compound A or a salt thereof, or a solvate thereof is effective in suppressing ballooning of hepatocytes, reducing Kupffer cell-positive area, and suppressing fat deposition in nonalcoholic steatohepatitis (NASH), severe symptoms of nonalcoholic fatty liver disease (NAFLD). The present invention provides useful bulk pharmaceuticals. Therefore, the present invention is useful in the pharmaceutical industry and has industrial applicability.

## Claims

1. (R)-2-[3-[[N-(benzoxazol-2-yl)-N-3-(4-methoxyphenoxy)propyl]aminomethyl]phenoxy]butyric acid or a salt thereof, or a solvate thereof for use in the prophylaxis and/or treatment of nonalcoholic fatty liver disease.

2. (R)-2-[3-[[N-(benzoxazol-2-yl)-N-3-(4-methoxyphenoxy)propyl]aminomethyl]phenoxy]butyric acid or a salt thereof, or a solvate thereof for use in the prophylaxis and/or treatment of nonalcoholic steatohepatitis.

## Patentansprüche

1. (R)-2-[3-[[N-(Benzoxazol-2-yl)-N-3-(4-methoxyphenoxy)propyl]aminomethyl]phenoxy]buttersäure oder ein Salz davon oder ein Solvat davon zur Verwendung bei der Prophylaxe und/oder Behandlung von nichtalkoholischer Fettlebererkrankung.

2. (R)-2-[3-[[N-(Benzoxazol-2-yl)-N-3-(4-methoxyphenoxy)propyl]aminomethyl]phenoxy]buttersäure oder ein Salz davon oder ein Solvat davon zur Verwendung bei der Prophylaxe und/oder Behandlung von nichtalkoholischer Steatohepatitis.

## Revendications

1. Acide (R)-2-[3-[[N-(benzoxazol-2-yl)-N-3-(4-méthoxyphénoxy)propyl]aminométhyl]phénoxy]butyrique ou un sel de celui ou un solvate de celui-ci pour l'utilisation dans la prophylaxie et/ou le traitement du stéatose hépatique non alcoolique.

2. Acide (R)-2-[3-[[N-(benzoxazol-2-yl)-N-3-(4-méthoxyphénoxy)propyl]aminométhyl]phénoxy]butyrique ou un sel de celui ou un solvate de celui-ci pour l'utilisation dans la prophylaxie et/ou le traitement de la stéatohépatite non alcoolique.
